# EUROPEAN PATENT SPECIFICATION

(11) **EP 2 457 216 B1**
(45) Date of publication and mention of the grant of the patent: **08.11.2017**
(21) Application number: 10731582.2
(22) Date of filing: 15.06.2010
(51) Int. Cl.: G06T 7/00, A61B 6/03

(54) **ANATOMY MODELING FOR TUMOR REGION OF INTEREST DEFINITION**
MODELLIERUNG DER ANATOMIE ZUR DEFINITION DES INTERESSIERENDEN TUMORBILDBEREICHS
MODÉLISATION ANATOMIQUE POUR DÉFINIR LA RÉGION D'INTERÊT D'UN TUMEUR

(30) Priority: 20.07.2009 US 226939 P
(43) Date of publication of application: 30.05.2012
(73) Proprietor: Koninklijke Philips N.V., 5656 AE Eindhoven (NL); Philips Intellectual Property & Standards GmbH, 20099 Hamburg (DE)
(72) Inventor: RENISCH, Steffen, NL-5656 AE Eindhoven (NL); OPFER, Roland, NL-5656 AE Eindhoven (NL)
(74) Representative: van Velzen, Maaike Mathilde
(86) International application number: PCT/IB2010/052689
(87) International publication number: WO 2011/010231

(56) References cited:
- US-A1- 2007 081 712
- US-A1- 2008 050 000
- HAN J ET AL: "Computer-aided evaluation of anatomical accuracy of image fusion between X-ray CT and SPECT" COMPUTERIZED MEDICAL IMAGING AND GRAPHICS, vol. 32, no. 5, 1 July 2008 (2008-07-01), pages 388-395, XP022686432 PERGAMON PRESS, NEW YORK, NY, US ISSN: 0895-6111 DOI: 10.1016/J.COMPMEDIMAG.2008.03.002 [retrieved on 2008-05-12]
- SCHILLACI ET AL: "Single-Photon Emission Computed Tomography/Computed Tomography in Abdominal Diseases" SEMINARS IN NUCLEAR MEDICINE, vol. 37, no. 1, 8 December 2006 (2006-12-08), pages 48-61, XP005748772 GRUNE AND STRATTON,, ORLANDO, FL, US ISSN: 0001-2998 DOI: 10.1053/J.SEMNUCLMED.2006.07.001
- HAIYING GUAN ET AL: "Automatic Hot Spot Detection and Segmentation in Whole Body FDG-PET Images" IEEE INTERNATIONAL CONFERENCE IMAGE PROCESSING, 1 October 2006 (2006-10-01), pages 85-88, XP031048579 ISBN: 978-1-4244-0480-3
- APARNA KANAKATTE ET AL: "A Pilot Study of Automatic Lung Tumor Segmentation from Positron Emission Tomography Images using Standard Uptake Values" COMPUTATIONAL INTELLIGENCE IN IMAGE AND SIGNAL PROCESSING, 1 April 2007 (2007-04-01), pages 363-368, XP031095582 ISBN: 978-1-4244-0707-1
- BLAFFERT T ET AL: "Hot spot detection, segmentation, and identification in PET images" PROCEEDINGS OF THE SPIE - THE INTERNATIONAL SOCIETY FOR OPTICAL ENGINEERING SPIE - THE INTERNATIONAL SOCIETY FOR OPTICAL ENGINEERING USA, vol. 6144, 2006, pages 614457-1-614457-8, XP002601427 ISSN: 0277-786X
- Hamarneh Ghasan, Abugharbieh Rafeef (Editors): "Proceedings of the First Workshop on Analysis of Functional Medical Images" 10 September 2008 (2008-09-10), , XP002601428 the whole document

## Description

The present application relates to diagnostic imaging systems and methods. It finds particular application in conjunction with Positron Emission Tomography (PET) muti-modality imaging systems and will be described with particular reference to hot spot detection of multi-focal diseases and lesion quantification.

Nuclear medicine imaging employs a source of radioactivity to image a patient. Typically, a radiopharmaceutical is injected into the patient. Radiopharmaceutical compounds contain a radioisotope that undergoes decay at a predictable rate and emits or causes the emission of gamma (γ) rays of a characteristic energy. One or more radiation detectors are placed adjacent to the patient to monitor and record emitted radiation. Sometimes, the detector is rotated or indexed around the patient to monitor the emitted radiation from a plurality of directions. Based on information such as detected position and energy, the radiopharmaceutical distribution in the body is determined and an image of the distribution is reconstructed to study the circulatory system, radiopharmaceutical uptake in selected organs or tissue, and the like.

Nuclear medicine imaging has been increasingly used in cancer imaging due to the success of the tracer [18F]-fluorodeoxyglucose (FDG). Focal areas of abnormally increased FDG uptake are known as hot spots and display as regions of high local intensity in PET images. The hot spots indicate a region of high metabolic activity, which may be a result of tumor lesions or other malignant processes. Accurate and reliable quantification of lesion activity in PET images is essential for patient staging as well as monitoring the response to treatment for many cancer types.

In order to assess the images, in the current clinical practice a clinician manually marks and segments lesions, which are assumed to be indicative of the entire disease. However, multi-focal diseases such as lymphoma and other diseases with lymph node involvement and metastases, the number of foci might be too large. Due to time constraints, usually three lead lesions are defined. However, indicative lesions are often obscured by non-pathological tracer uptake of normal anatomical structures. In the case of FDG-PET, normally functioning organs with high metabolic activity such as the heart, brain, bladder, liver, K, or the like often show FDG uptake unrelated to cancer. Additionally, Partial volume effects (PVE) pose another problem for identifying tumor lesions in PET images.

Often, the PET images are combined with the computed tomography (CT) images, magnetic resonance (MR) images, or other images of the same anatomical region.

US 2007/0081712 A1 discloses a system and method for whole-body landmark detection, segmentation, and change quantification in single-mode and multimode medical images. It includes methods and systems for discriminative learning-based framework that uses databases to guide the modeling and detection of landmarks in whole-body CT, MRI or PET images, and an automatic method for determining the intensity threshold for hot-spot segmentation in PET images.

A further device and method that make use of hot spot detection, segmentation and identification in PET and SPECT images is known from US 2008/0050000 A1.

Han, J. et al.: "Computer-aided evaluation of anatomical accuracy of image fusion between X-ray CT and SPECT", Computerized Medical Imaging and Graphics, Pergamon Press, New York, Vol. 32, No. 5, 1 July 2008, pages 388-395 discloses a method for hybrid scanning by combining the performance of single photon emission computed tomography (SPECT) and X-ray computed tomography (CT) in one imaging session.

The present application provides a new and improved PET imaging system and method which overcomes the above-referenced problems and others.

In accordance with one aspect, a hot spot detection system includes a segmentation unit which segments an anatomical first image representation into regions corresponding to anatomical structures of a subject and a hot spot detection unit which detects regions of high uptake from a functional second image representation. A classification unit classifies the regions of high tracer uptake according to their position relative to the anatomical structures segmented from the anatomical first image representation. An uptake unit examines segmented regions of the first image representation for at least one of homogeneity and uptake to identify normal and abnormal regions. A suppression unit suppresses regions of high tracer uptake in the functional second image representation based on the results of the classification unit, wherein said regions being suppressed correspond to anatomical structures identified as normal. An identification unit identifies unsuppressed regions of high uptake as one of potential lesion and non-potential lesion.

A normalization unit compares the metabolic activity of an unsuppressed high intensity region with the metabolic activity of regions identified as normal by the uptake unit.

In accordance with another aspect, a method for diagnostic imaging includes segmenting an anatomical first image representation into regions corresponding to anatomical structures and detecting from a functional second image representation a plurality of regions of high tracer uptake. The regions of high tracer uptake are classified according to their position relative to the anatomical structures segmented from the anatomical first image representation. The segmented regions of the first image representation are then examined for at least one of homogeneity and uptake to identify normal and abnormal regions. Regions of high uptake in the functional second image representation that correspond with one or more segmented regions of the segmented anatomical first image representation are suppressed, wherein the regions being suppressed correspond to anatomical structures identified as normal. And, unsuppressed regions of high uptake are indentified as one of potential and non-potential lesions. The metabolic activity of an unsuppressed high intensity region is compared with the metabolic activity of regions identified as normal.

In accordance with a third aspect, anatomical regions identified in the anatomical first image representation could be carried over to the functional second image representation in order to delineate anatomical structures there. The tracer uptake in these structures could be used as a reference for the quantification of the hot spots, e.g. the relative activity of a hot spot compared with that of normal liver tissue could be computed.

One advantage relies in that automatic detection and quantification of lesions is improved.

Another advantage relies in that time of identification of lesions is improved

Still further advantages of the present invention will be appreciated to those of ordinary skill in the art upon reading and understand the following detailed description.

The invention may take form in various components and arrangements of components, and in various steps and arrangements of steps. The drawings are only for purposes of illustrating the preferred embodiments and are not to be construed as limiting the invention.
FIGURE 1 diagrammatically shows a diagnostic imaging system employing a hot spot detection system;
FIGURE 2 diagrammatically shows a hot spot detection system;
FIGURE 3A illustrates regions of high intensity in a functional image representation;
FIGURE 3B illustrates anatomical structures segmented in an anatomical image representation;
FIGURE 3C illustrates regions of high intensity after anatomical structures are suppressed;
FIGURE 4 illustrates a watershed algorithm; and
FIGURE 5 is a flow chart depicting a method for detecting regions of high uptake.

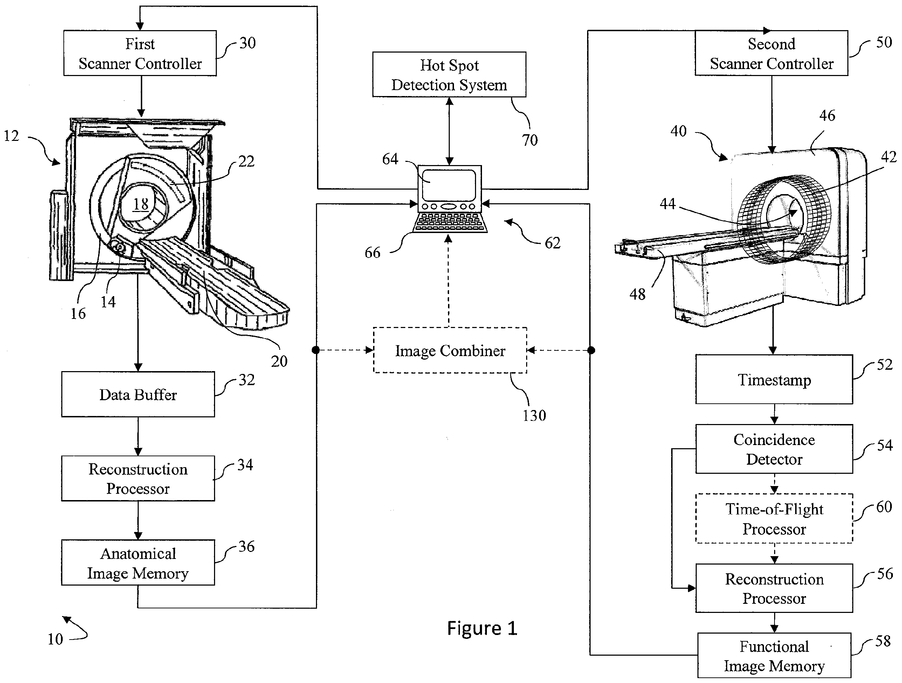

With reference to FIG. 1, a diagnostic system **10** includes a first imaging system, a diagnostic imaging scanner **12** such as a computed tomography (CT) imaging scanner, an MRI scanner, or the like for obtaining anatomical diagnostic images. In the illustrated embodiment, the diagnostic imaging scanner **12** includes an x-ray source **14** mounted on a rotating gantry **16.** The x-ray source **14** produces x-rays passing through an examination region **18,** where they interact with a target area of a subject (not shown) supported by a support **20** which positions the target area within the examination region **18.** An x-ray detector array **22** is arranged to receive the x-ray beam after it passes through the examination region **18** where the x-rays interact with and are partially absorbed by the subject. The detected x-rays therefore include absorption information relating to the subject.

The CT scanner **12** is operated by a controller **30** to perform selected imaging sequences of a selected target area. The imaging sequences acquire diagnostic imaging data of the target area. The diagnostic imaging data is stored in a data buffer **32.** A reconstruction processor **34** reconstructs 3D image representations from the acquired imaging data, and the reconstructed image representations are stored in a diagnostic anatomical image memory **36.**

The diagnostic system **10** includes a second imaging system, particularly a functional imaging scanner **40** such as a PET scanner, SPECT scanner, or the like for obtaining functional images. In the illustrated embodiment, the functional imaging scanner **40,** which is illustrated as a PET scanner, includes a plurality of radiation detector modules **42** oriented to receive γ radiation from an imaging region **44.** The radiation detector modules **42** are arranged in several adjacent rings along an axial direction as depicted; however, other arrangements of radiation detector modules can be used. The radiation detector modules **42** are typically housed within a housing **46** of the tomography scanner **40** and thus are not visible from the outside. Typically, each ring is comprised of hundreds or thousands of radiation detector modules **42.** The functional imaging scanner **40** includes a subject support **48** for positioning an object or a human patient in the imaging region **44.** The support **48** is linearly movable in the axial direction generally transverse to the rings of the radiation detector modules **42** to facilitate acquisition of three-dimensional imaging data over an extended axial distance.

The PET scanner **40** is operated by a controller **50** to perform selected imaging sequences of a selected target area. Typically, an object or patient to be imaged is injected with one or more radiopharmaceutical or radioisotope tracers and placed in the examination region **44** supported by the support **48.** Examples of such tracers are 18F FDG, C-11, Tc-99m, Ga67, and In-111. The presence of the tracer within the object produces emission radiation from the object. Radiation events are detected by the detector modules **42** around the examination region **44.** A time stamp is associated with each detected radiation event by a time stamp circuit **52.** A coincidence detector **54** determines coincident pairs of γ rays and the line of responses (LOR) defined by each coincident pair of γ rays based on differences in detection time of the coincidence pairs and the known diameter of the field of view. A reconstruction processor **56** reconstructs all the LORs into an image representation which is stored in a functional image memory **58.** Optionally, a time-of-flight processor **60** localizes each radiation event by deriving time-of-flight information from the timestamps for each LOR. A workstation or graphic user interface **62** includes a display device **64** and a user input device **66** which a clinician can use to select scanning sequences and protocols, display image data, and the like.

Although the anatomical and functional scanners are illustrated separate, it is to be appreciated that they can be combined. In one embodiment, the anatomical and functional scanners are connected contiguous to each other with the central axes of their imaging regions aligned. A common support moves the object or patient sequentially through the imaging regions. In another embodiment, the anatomical and functional scanners share a common housing and a common imaging region. For example, the PET detectors can surround the imaging volume and an MRI magnet and gradient magnetic field coils and RF transmit and/or receive coils can be disposed around, inside of, and up or downstream from the ring of PET detectors. Similar combined PET/CT, SPECT/CT, SPECT/MRI, PET/ultrasound, etc. scanners are also contemplated.

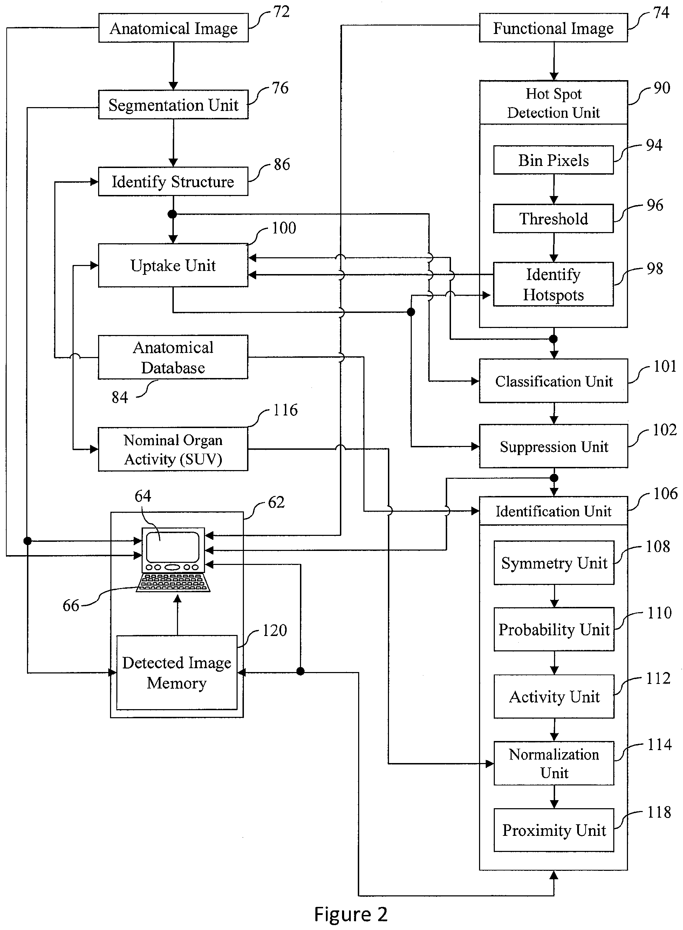

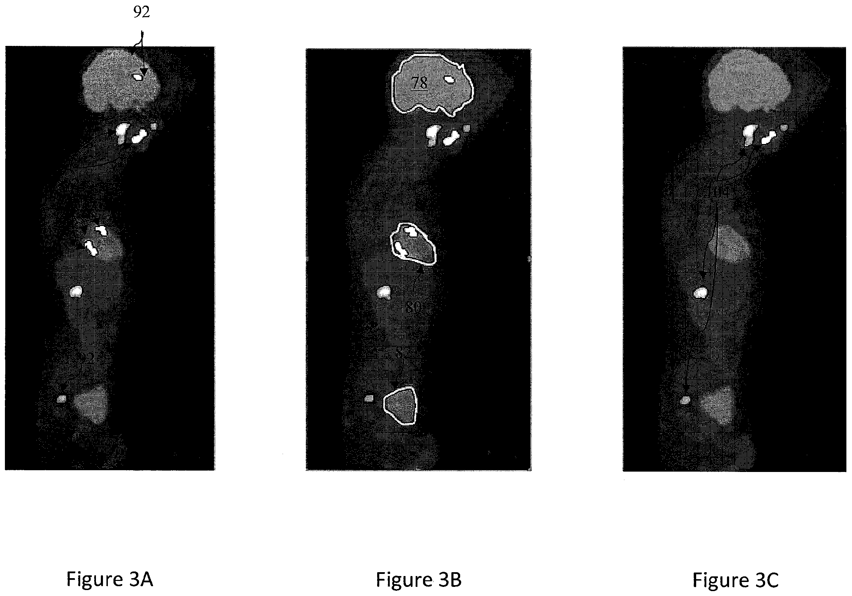

Continuing with reference to FIGURE 1 and further reference to FIGURES 2 and 3B, the diagnostic system **10** includes a hot spot detection system **70** for automatic detection of a region of interest (ROI) pertaining to a lesion and automatic quantification of metabolic activity in detected lesions based on anatomical images **72** from the anatomical image memory **36** and functional images **74** from the functional image memory **58.** A segmentation unit **76** segments the anatomical first image representation **72** into regions which correspond to anatomical structures, particularly anatomical structures with high radiopharmaceutical tracer uptake which may obscure potential lesions of interest. In the case of FDG-PET, the brain **78,** the heart **80,** and the bladder **82** are organs of FIGURE 3B which, when functioning normally, are examples of anatomical structures which often show high uptake unrelated to cancer. Other organs with high uptake include the kidneys and liver which are also contemplated for segmented anatomical structures. Segmentation can also be helpful for accurately determining a location of the hot spots relative to the patient's anatomy.

While the anatomical image representation **72** and the functional image representation **74** have been so far exemplified as different types of image representations, they can hypothetically be identical. They can be both PET, SPECT, CT, MR, or the like image representations.

The segmentation unit **76** is capable of employing different types of segmentation methods. For example, the segmentation unit **76** can employ a model-based segmentation in which the central assumption is that the anatomical structures of interest have, to some extent, relatively consistent forms of geometry and position across patients. A library of three-dimensional anatomical structure models explaining the shape, geometrical location, size, and variations thereof are defined in an anatomical database **84** prior to the segmentation. During segmentation, the models act as templates to identify **86** and define the boundary of the structure of interest. It is to be appreciated, however, that other segmentation methods such as clustering, edge detection, region growing, principle components analysis, neural network, and the like are also contemplated.

The segmentation unit **76** can also employ an atlas of normal anatomical structures which is mapped to the actual anatomical image. In such an embodiment, the atlas includes the anatomical database **84.**

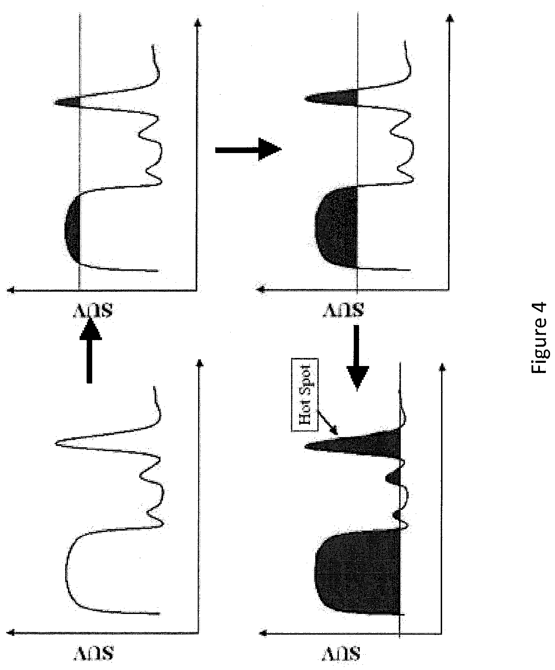

With continuing reference to FIGURE 2 and further reference to FIGURES 3A and 4, a hot spot detection unit **90** detects from the functional second image representation **74** regions of high intensity **92,** depicted in FIGURE 3A. The regions of high intensity **92,** generally referred to as hot spots, are regions in the functional second image representation that indicate high metabolic activity, which potentially can be caused by tumor growth or by other malignant processes. These regions of high intensity also include normal functioning structures/organs such as the heart, bladder, kidney, liver, and brain. The regions of high intensity **92** are detected, for example, by a watershed algorithm. A bin sorting unit, processor, or algorithm **94** sorts all the voxels of the second image representation **74** according to grayscale values. Basins with the highest grayscale values are effectively flooded, as depicted in FIGURE 4. For each basin, a measure of the compactness of the basin-region and the amplitude between the highest and the lowest grayscale values within the basin is computed. Basins which exceed a certain threshold **96** are highlighted as regions of high intensity **92** with a color overlay. An identification unit, processor, or algorithm **98** uses anatomical information, such as size, shape, location, or the like, from the anatomical database **84** to identify the regions with high tracer uptake, for example as a salivary gland. It should be appreciated that other detection algorithms for detecting regions of high intensity are also contemplated.

With continuing reference to FIGURE 2, an uptake unit, processor, or algorithm **100** examines the segmented regions **78, 80, 82** of the first image representation to determine whether the anatomical structure is functioning normally or abnormally based on, for example, at least one of homogeneity and uptake. The uptake unit **100** correlates metabolic activity from the functional image representation **74** corresponding to the segmented regions of the anatomical image representation **72** to determine an uptake value, particularly a standardized uptake value (SUV), a measure of the concentration of the radiopharmaceutical tracer. A segmented region with a consistent uptake value throughout the segmented region and an average uptake value consistent with a predefined level is considered normal.

A classification unit, processor, or algorithm **101** classifies the regions of high tracer uptake according to their position relative to the anatomical structures segmented from the anatomical first image representation. A suppression unit, processor, or algorithm **102** uses the results of the classification unit to suppress the regions of high intensity **92** in the functional second image representation **74.** As previously noted, regions of high intensity that correspond to normally functioning anatomical structures may obscure potentially hazardous lesions. To improve detection of potential lesions, normally functioning anatomical structures are suppressed from the functional second image representation while unsuppressed regions **104,** shown in FIGURE 3C, of high intensity are further analyzed to determine if they are plausible lesions. It should also be noted that suppressing abnormally functional anatomical structures is also contemplated.

An identification unit, processor, or algorithm **106** is configured to calculate metrics corresponding to the unsuppressed regions. The metrics include, but are not limited to, total tumor burden, glycolytic volume, SUV, average activity, maximum activity, minimum activity, homogeneity, and the like. In addition to calculating metrics, the quantification unit includes a number of modules that perform various checks on the unsuppressed regions **104** to determine if they are lesions. For example, a symmetry unit, processor, or algorithm **108** determines symmetric pairs of the high intensity regions of the second and/or first image representation that correspond to paired anatomical structures. For example, if a high intensity region is identified, by the identification unit **98,** as a salivary gland, the symmetry unit searches for the corresponding salivary gland on the other side of the patient. Activity patterns between the symmetric pairs are determined by an activity unit, processor, or algorithm **110.** If an asymmetry of metabolic activity exists between the symmetric pair, the unsuppressed high intensity region **104** is identified as a potential lesion.

In another embodiment, the results of the identification unit **106** are visualized on the display device **64.** The user is asked to confirm or edit the selected hot spots, i.e. high uptake regions, using the input device **66** before a summary report is generated.

The quantification unit also includes a probability unit, processor, or algorithm **112** which determines a probability for a high intensity region in the second image representation whether a lymph node is present. Assessing lymph nodes, such as axillary or mediastinal, is a critical component in determining whether a tumor is likely to have metastasized, which dictates the therapeutic options for cancer patients. A normalization unit, processor, or algorithm **114** compares the metabolic activity of an unsuppressed high intensity region with normally functioning structures, e.g. those identified by the uptake unit **100.** For example, metabolic activity of a potential lesion is commonly compared to a standard uptake value determined by a nominal activity unit, processor, or algorithm **116.** The nominal activity unit is also capable of determining total tumor burden, total glycolytic volume, or the like. The metabolic activity of the liver can be used as a reference for comparison. Since the liver is segmented and indentified by the segmentation unit **76,** an unsuppressed high intensity region can easily be compared to the uptake in the liver. Previously, a clinician had to manually delineate a region of the liver on a workstation which is time consuming and highly subjective. The anatomical regions identified in the anatomical first image representation **72** can be carried over to the functional second image representation **74** in order to delineate anatomical structures in the second image representation **74.** The tracer uptake in these structures could be used as a reference for the quantification of the hot spots, e.g. the relative activity of a hot spot compared with that of normal liver tissue could be computed.

The quantification unit also includes a proximity unit **118** which identifies high intensity regions, based on models stored in the anatomy database **84,** in close proximity to suppressed regions. The proximity units makes use of anatomical information, provided by the anatomical database **84,** to determine where high intensity regions are likely to occur and not occur. This can be translated to the suppression unit and/or segmentation unit to adjust suppressed regions include or exclude regions. For example, if a lymph node is a located close to the heart, *a priori* knowledge of heart models and lymph node models is used to ensure that the lymph node has not been included in the segmented heart, thus suppressed in the second image representation.

The segmented and unsegmented anatomical images, the function images, the functional image with high uptake organs suppressed, in image or map of quantified hot spots, and superimposed combinations thereof are stored in a detected image memory **120** of a workstation or graphic user interface **62.**

With returning reference to FIGURE 1, an optional image combiner **130** combines the anatomical first image representation and the functional second image representation into a combined image for concurrent display. For example, the images can be superimposed in different colors, the outline of the functional second image representation hotspots can be superimposed on the first image representation, the outline of the segmented anatomical structures of the anatomical first image representation can be superimposed on the functional second image representation, the first and second image representations can be displayed side by side with a common scale, or the like.

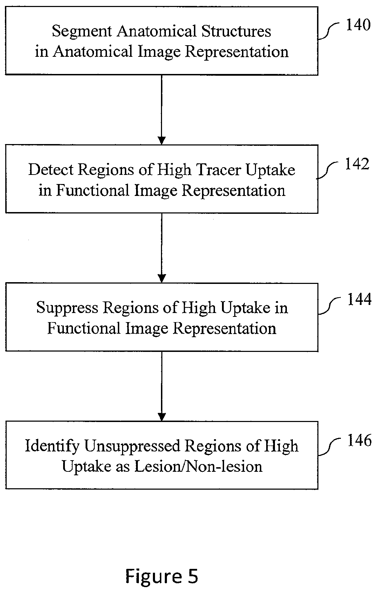

With reference to FIGURE 5, a method for diagnostic imaging includes segmenting **140** an anatomical first image representation into regions corresponding to anatomical structures and detecting **142** from a functional second image representation a plurality of regions of high tracer uptake. Regions of high uptake in the functional second image representation that correspond with one or more segmented regions of the segmented anatomical first image representation are suppressed **144.** And, unsuppressed regions of high uptake are indentified **146** as one of potential and non-potential lesions.

The invention has been described with reference to the preferred embodiments. Modifications and alterations may occur to others upon reading and understanding the preceding detailed description. It is intended that the invention be constructed as comprising all such modifications and alterations insofar as they come within the scope of the appended claims or the equivalents thereof.

## Claims

1. A hot spot detection system (70), including:
a segmentation unit (76) configured to segment an anatomical first image representation (72) into regions corresponding to anatomical structures of a subject;
a hot spot detection unit (90) configured to detect regions of high tracer uptake from a functional second image representation (74);
a classification unit (101) configured to classify the regions of high tracer uptake according to their position relative to the anatomical structures segmented from the anatomical first image representation (72);
an uptake unit (100) configured to examine segmented regions of the first image representation (72) for at least one of homogeneity and uptake to identify normal and abnormal regions;
a suppression unit (102) configured to suppress regions of high tracer uptake in the functional second image representation (74) based on results of the classification unit, wherein said regions being suppressed correspond to anatomical structures identified as normal; and
an identification unit (106) configured to identify unsuppressed regions of high uptake as one of potential lesion and non-potential lesion,
**characterized in that** the system further includes:
a normalization unit (114) configured to compare the metabolic activity of an unsuppressed high intensity region with the metabolic activity of regions identified as normal by the uptake unit (100).

2. The hot spot detection system (70) according to claim 1, wherein the segmentation unit (76) is configured to identify and to segment the anatomical structure based on a priori information according to at least one of shape, size, and geometrical location of the anatomical structure.

3. The hot spot detection system (70) according either one of claim 1 and 2, further including:
a probability unit (112) configured to determine a probability of the high uptake regions of the functional image representation corresponding to a lymph node.

4. The hot spot detection system (70) according to any one of claims 1-3, further including:
a symmetry unit (108) configured to determine symmetric pairs of the high uptake regions of the functional image representation (74) that correspond to paired anatomical structures; and
an activity unit (110) configured to compare metabolic activity between the determined symmetric pairs of high uptake regions to identify asymmetry in metabolic activity.

5. The hot spot detection system (70) according to claim any one of claims 1-4, further comprising:
a nominal activity unit (116) configured to determine a metric for the unsuppressed high intensity regions based on any one of total tumor burden, total glcolytic volume, and standardized uptake value.

6. The hot spot detection system (70) according to claim 5, wherein the nominal activity unit (116) is configured to determine a nominal metabolic activity in a selected segmented region.

7. The hot spot detection system (70) according to claim 6, wherein the selected segmented region corresponds to a liver.

8. The hot spot detection system (70) according to any one of claims 1-7, further including:
a workstation (62) configured to outline the detected regions of high uptake with a color overlay and to display the detected regions of high uptake on a display device (64).

9. A diagnostic imaging system (10), comprising:
an anatomical image scanner (12) configured to generate an anatomical first image representation;
a positron emission tomography (PET) scanner (40) configured to generate a functional second image representation; and
a hot spot detection system (70) according to any one of claims 1-8.

10. A method for diagnostic imaging, including:
segmenting a anatomical first image representation (72) into regions corresponding to anatomical structures;
detecting from a functional second image representation (74) a plurality of regions of high tracer uptake;
classifying the regions of high tracer uptake according to their position relative to the anatomical structures segmented from the anatomical first image representation (72);
examining segmented regions of the first image representation (72) for at least one of homogeneity and uptake to identify normal and abnormal regions
suppressing regions of high uptake in the functional second image representation (74) based on the classification of the high tracer uptake regions, wherein the regions being suppressed correspond to anatomical structures identified as normal; and
identifying unsuppressed regions of high uptake as one of potential lesion and non-potential lesion
**characterized by** the step of:
comparing the metabolic activity of an unsuppressed high intensity region with the metabolic activity of regions identified as normal.

11. The method according to claim 10, further including:
determining symmetric pairs of the high uptake regions of the second image representation that correspond to paired anatomical structures based on anatomical information; and
comparing metabolic activity between the determined symmetric pairs of high uptake regions to identify asymmetry in metabolic activity.

12. The method according to either one of claims 10 and 11, further including:
normalizing metabolic activity levels of the unsuppressed regions of high uptake with a nominal intensity in a selected segmented region.

13. The method according to any one of claims 10-12, wherein segmenting the anatomical structure is based on a priori information according to at least one of shape, size, and geometrical location of the anatomical structure.

14. The method according to any one of claims 10-13, wherein the functional image representation (74) is generated by a PET imaging scanner (40).

15. The method according to any one of claims 10-14, further comprising the step of displaying the unsuppressed regions of high uptake.

## Patentansprüche

1. Hot-Spot-Detektionssystem (70), umfassend:
eine Segmentierungseinheit (76), die dafür konfiguriert ist, eine anatomische erste Bilddarstellung (72) in Regionen zu segmentieren, die anatomischen Strukturen eines Patienten entsprechen;
eine Hot-Spot-Detektionseinheit (90), die dafür konfiguriert ist, Regionen mit hoher Traceraufnahme aus einer funktionalen zweiten Bilddarstellung (74) zu detektieren;
eine Klassifizierungseinheit (101), die dafür konfiguriert ist, die Regionen mit hoher Traceraufnahme nach ihrer Position in Bezug auf die anatomischen Strukturen zu klassifizieren, die aus der anatomischen ersten Bilddarstellung (72) segmentiert wurden;
eine Aufnahmeeinheit (100), die dafür konfiguriert ist, segmentierte Regionen der ersten Bilddarstellung (72) auf mindestens entweder Homogenität oder Aufnahme zu untersuchen, um normale und abnormale Regionen zu identifizieren;
eine Unterdrückungseinheit (102), die dafür konfiguriert ist, Regionen mit hoher Traceraufnahme in der funktionalen zweiten Bilddarstellung (74) basierend auf Ergebnissen der Klassifizierungseinheit zu unterdrücken, wobei die genannten unterdrückten Regionen anatomischen Strukturen entsprechen, die als normal identifiziert wurden; und
eine Identifizierungseinheit (106), die dafür konfiguriert ist, nichtunterdrückte Regionen mit hoher Aufnahme als potentielle Läsion oder nicht-potenzielle Läsion zu identifizieren,
**dadurch gekennzeichnet, dass** das System weiterhin Folgendes umfasst:
eine Normalisierungseinheit (114), die dafür konfiguriert ist, die Stoffwechselaktivität einer nicht-unterdrückten Region mit hoher Intensität mit der Stoffwechselaktivität von Regionen zu vergleichen, die durch die Aufnahmeeinheit (100) als normal identifiziert wurden.

2. Hot-Spot-Detektionssystem (70) nach Anspruch 1, wobei die Segmentierungseinheit (76) dafür konfiguriert ist, die anatomische Struktur basierend auf a priori Informationen entsprechend mindestens entweder der Form, Größe oder geometrischen Lage der anatomischen Struktur zu identifizieren und zu segmentieren.

3. Hot-Spot-Detektionssystem (70) nach einem der Ansprüche 1 und 2, weiterhin umfassend:
eine Wahrscheinlichkeitseinheit (112), die dafür konfiguriert ist, eine Wahrscheinlichkeit zu ermitteln, dass die Regionen mit hoher Aufnahme in der funktionalen Bilddarstellung einem Lymphknoten entsprechen.

4. Hot-Spot-Detektionssystem (70) nach einem der Ansprüche 1 bis 3, weiterhin umfassend:
eine Symmetrieeinheit (108), die dafür konfiguriert ist, symmetrische Paare von Regionen mit hoher Aufnahme in der funktionalen Bilddarstellung (74) zu ermitteln, die gepaarten anatomischen Strukturen entsprechen; und
eine Aktivitätseinheit (110), die dafür konfiguriert ist, die Stoffwechselaktivität der einzelnen ermittelten symmetrischen Paare von Regionen mit hoher Aufnahme zu vergleichen, um eine Asymmetrie in der Stoffwechselaktivität zu identifizieren.

5. Hot-Spot-Detektionssystem (70) nach einem der Ansprüche 1 bis 4, weiterhin umfassend:
eine Nominalaktivitätseinheit (116), die dafür konfiguriert ist, ein Maß für die nicht-unterdrückten Regionen hoher Intensität basierend auf einem von der gesamten Tumorlast, dem gesamten glykolytischem Volumen und dem standardisierten Aufnahmewert zu ermitteln.

6. Hot-Spot-Detektionssystem (70) nach Anspruch 5, wobei die Nominalaktivitätseinheit (116) dafür konfiguriert ist, eine nominale Stoffwechselaktivität in einer ausgewählten segmentierten Region zu ermitteln.

7. Hot-Spot-Detektionssystem (70) nach Anspruch 6, wobei die ausgewählte segmentierte Region einer Leber entspricht.

8. Hot-Spot-Detektionssystem (70) nach einem der Ansprüche 1 bis 7, weiterhin umfassend:
eine Workstation (62), die dafür konfiguriert ist, die detektierten Regionen mit hoher Aufnahme mit einer Farbüberlagerung darzustellen und die detektierten Regionen mit hoher Aufnahme auf einer Anzeigevorrichtung (64) anzuzeigen.

9. Diagnostisches Bildgebungssystem (10), umfassend:
einen anatomischen Bildscanner (12), der dafür konfiguriert ist, eine anatomische erste Bilddarstellung zu erzeugen;
einen Positronen-Emissions-Tomographie- (PET) Scanner (40), der dafür konfiguriert ist, eine funktionale zweite Bilddarstellung zu erzeugen; und
ein Hot-Spot-Detektionssystem (70) nach einem der Ansprüche 1 bis 8.

10. Verfahren zur diagnostischen Bilddarstellung, umfassend:
Segmentieren einer anatomischen ersten Bilddarstellung (72) in Regionen, die anatomischen Strukturen entsprechen;
Detektieren einer Vielzahl von Regionen mit hoher Traceraufnahme aus einer funktionalen zweiten Bilddarstellung (74);
Klassifizieren der Regionen mit hoher Traceraufnahme nach ihrer Position in Bezug auf die anatomischen Strukturen, die aus der anatomischen ersten Bilddarstellung (72) segmentiert wurden;
Untersuchen der segmentierten Regionen der ersten Bilddarstellung (72) auf mindestens entweder Homogenität oder Aufnahme, um normale und abnormale Regionen zu identifizieren;
Unterdrücken von Regionen mit hoher Traceraufnahme in der funktionalen zweiten Bilddarstellung (74) basierend auf der Klassifikation von Regionen mit hoher Traceraufnahme, wobei die unterdrückten Regionen anatomischen Strukturen entsprechen, die als normal identifiziert wurden; und
Identifizieren von nicht-unterdrückten Regionen mit hoher Aufnahme als potentielle Läsion oder nicht-potenzielle Läsion,
**gekennzeichnet durch** den folgenden Schritt:
Vergleichen der Stoffwechselaktivität einer nicht-unterdrückten Region mit hoher Intensität mit der Stoffwechselaktivität von Regionen, die als normal identifiziert wurden.

11. Verfahren nach Anspruch 10, weiterhin umfassend:
Ermitteln von symmetrischen Paaren von Regionen mit hoher Aufnahme in der zweiten Bilddarstellung, die gepaarten anatomischen Strukturen entsprechen, basierend auf anatomischen Informationen; und
Vergleichen der Stoffwechselaktivität der einzelnen ermittelten symmetrischen Paare von Regionen mit hoher Aufnahme, um eine Asymmetrie in der Stoffwechselaktivität zu identifizieren.

12. Verfahren nach einem der Ansprüche 10 und 11, weiterhin umfassend:
Normalisieren von Stoffwechselaktivitätsniveaus der nicht-unterdrückten Regionen hoher Aufnahme mit einer nominalen Intensität in einer ausgewählten segmentierten Region.

13. Verfahren nach einem der Ansprüche 10 bis 12, wobei das Segmentieren der anatomischen Struktur auf a priori Informationen entsprechend mindestens entweder der Form, Größe oder geometrischen Lage der anatomischen Struktur basiert.

14. Verfahren nach einem der Ansprüche 10 bis 13, wobei die funktionale Bilddarstellung (74) durch einen PET-Bildgebungs-Scanner (40) erzeugt wird.

15. Verfahren nach einem der Ansprüche 10 bis 14, weiterhin umfassend den Schritt des Anzeigens der nicht-unterdrückten Regionen mit hoher Aufnahme.

## Revendications

1. Système de détection de point chaud (70), comprenant :
une unité de segmentation (76) conçue pour segmenter une première représentation d'image anatomique (72) dans des régions correspondant à des structures anatomiques d'un sujet ;
une unité de détection de point chaud (90) conçue pour détecter des régions d'absorption élevée de traceur à partir d'une seconde représentation d'image fonctionnelle (74) ;
une unité de classement (101) conçue pour classer les régions d'absorption élevée de traceur en fonction de leur position par rapport aux structures anatomiques segmentées à partir de la première représentation d'image anatomique (72) ;
une unité d'absorption (100) conçue pour examiner les régions segmentées de la première représentation d'image (72) pour au moins une parmi l'homogénéité et l'absorption afin d'identifier les régions normales et anormales ;
une unité de suppression (102) conçue pour supprimer les régions d'absorption élevée de traceur dans la seconde représentation d'image fonctionnelle (74) sur la base des résultats de l'unité de classement, dans lequel lesdites régions supprimées correspondent à des structures anatomiques identifiées comme normales ; et
une unité d'identification (106) conçue pour identifier les régions non supprimées d'absorption élevée comme des régions de lésion potentielle et de lésion non potentielle,
**caractérisé en ce que** le système comprend en outre :
une unité de normalisation (114) conçue pour comparer l'activité métabolique d'une région à intensité élevée non supprimée à l'activité métabolique de régions identifiées comme normales par l'unité d'absorption (100).

2. Système de détection de point chaud (70) selon la revendication 1, dans lequel l'unité de segmentation (76) est conçue pour identifier et pour segmenter la structure anatomique sur la base d'informations empiriques en fonction d'au moins un parmi la forme, la taille, et l'emplacement géométrique de la structure anatomique.

3. Système de détection de point chaud (70) selon l'une quelconque des revendications 1 et 2, comprenant en outre :
une unité de probabilité (112) conçue pour déterminer une probabilité des régions d'absorption élevée de la représentation d'image fonctionnelle correspondant à un noeud lymphoïde.

4. Système de détection de point chaud (70) selon l'une quelconque des revendications 1 à 3, comprenant en outre :
une unité de symétrie (108) conçue pour déterminer des paires symétriques des régions d'absorption élevée de la représentation d'image fonctionnelle (74) qui correspondent à des structures anatomiques appariées ; et
une unité d'activité (110) conçue pour comparer l'activité métabolique entre les paires symétriques déterminées de régions d'absorption élevée afin d'identifier une asymétrie dans l'activité métabolique.

5. Système de détection de point chaud (70) selon l'une quelconque des revendications 1 à 4, comprenant en outre :
une unité d'activité nominale (116) conçue pour déterminer une mesure métrique pour les régions à intensité élevée non supprimée sur la base de l'un quelconque parmi une charge tumorale totale, un volume glycolytique total et une valeur d'absorption standardisée.

6. Système de détection de point chaud (70) selon la revendication 5, dans lequel l'unité d'activité nominale (116) est conçue pour déterminer une activité métabolique nominale dans une région segmentée sélectionnée.

7. Système de détection de point chaud (70) selon la revendication 6, dans lequel la région segmentée sélectionnée correspond à un foie.

8. Système de détection de point chaud (70) selon l'une quelconque des revendications 1 à 7, comprenant en outre :
une station de travail (62) conçue pour tracer les contours des régions détectées d'absorption élevée avec une superposition de couleur et pour afficher les régions détectées d'absorption élevée sur un dispositif d'affichage (64).

9. Système d'imagerie diagnostique (10) comprenant :
un dispositif de balayage d'image anatomique (12) conçu pour générer une première représentation d'image anatomique ;
un tomographe par émission de positrons (PET) (40) conçu pour générer une seconde représentation d'image fonctionnelle ; et
un système de détection de point chaud (70) selon l'une quelconque des revendications 1 à 8.

10. Procédé d'imagerie diagnostique, comprenant :
la segmentation d'une première représentation d'image anatomique (72) dans des régions correspondant à des structures anatomiques ;
la détection à partir d'une seconde représentation d'image fonctionnelle (74) d'une pluralité de régions d'absorption élevée de traceur ;
le classement des régions d'absorption élevée de traceur en fonction de leur position par rapport aux structures anatomiques segmentées à partir de la première représentation d'image anatomique (72) ;
l'examen des régions segmentées de la première représentation d'image (72) pour au moins une parmi l'homogénéité et l'absorption afin d'identifier les régions normales et anormales ;
la suppression des régions d'absorption élevée dans la seconde représentation d'image fonctionnelle (74) sur la base du classement des régions d'absorption élevée de traceur, dans lequel les régions supprimées correspondent à des structures anatomiques identifiées comme normales ; et
l'identification des régions non supprimées d'absorption élevée comme des régions de lésion potentielle et de lésion non potentielle,
**caractérisé par** l'étape suivante :
la comparaison de l'activité métabolique d'une région à intensité élevée non supprimée à l'activité métabolique de régions identifiées comme normales.

11. Procédé selon la revendication 10, comprenant en outre :
la détermination de paires symétriques des régions d'absorption élevée de la seconde représentation d'image qui correspondent à des structures anatomiques appariées sur la base d'informations anatomiques ; et
la comparaison de l'activité métabolique entre les paires symétriques déterminées de régions d'absorption élevée afin d'identifier une asymétrie dans l'activité métabolique.

12. Procédé selon l'une quelconque des revendications 10 et 11, comprenant en outre :
la normalisation des niveaux d'activité métabolique des régions non supprimées d'absorption élevée par rapport à une intensité nominale dans une région segmentée sélectionnée.

13. Procédé selon l'une quelconque des revendications 10 à 12, dans lequel la segmentation de la structure anatomique est basée sur des informations empiriques en fonction d'au moins un parmi la forme, la taille, et l'emplacement géométrique de la structure anatomique.

14. Procédé selon l'une quelconque des revendications 10 à 13, dans lequel la représentation d'image fonctionnelle (74) est générée par un tomographe par émission de positrons (40).

15. Procédé selon l'une quelconque des revendications 10 à 14, comprenant en outre l'étape d'affichage des régions non supprimées d'absorption élevée.
